# EUROPEAN PATENT SPECIFICATION

(11) **EP 4 590 369 B1**
(45) Date of publication and mention of the grant of the patent: **10.06.2026**
(21) Application number: 23772876.1
(22) Date of filing: 19.09.2023
(51) Int. Cl.: A61M 5/32, A61M 5/34

(54) **TIP CAP FOR A MEDICAL INJECTION DEVICE**
SPITZENKAPPE FÜR EINE MEDIZINISCHE INJEKTIONSVORRICHTUNG
CAPUCHON DE POINTE POUR DISPOSITIF D'INJECTION MÉDICAL

(30) Priority: 19.09.2022 EP 22306372
(43) Date of publication of application: 30.07.2025
(73) Proprietor: Becton Dickinson France, 38800 Le Pont de Claix (FR)
(72) Inventor: CIBOULET, Antoine, 38000 Grenoble (FR); OZTURK, Senturk, 38130 Echirolles (FR)
(74) Representative: Regimbeau
(86) International application number: PCT/EP2023/075829
(87) International publication number: WO 2024/061924

(56) References cited:
- US-A1- 2018 256 880
- US-A1- 2019 046 736
- US-A1- 2022 080 129

## Description

### TECHNICAL FIELD

The disclosure relates to a tip cap for a medical device. The disclosure further relates to a tip cap assembly and medical injection device comprising said tip cap assembly.

### TECHNICAL BACKGROUND

In the present text, the distal end of a medical injection device must be understood as meaning the end furthest from the hand of the user and the proximal end must be understood as meaning the end closest to the hand of the user, with reference to the injection system intended to be used with said medical injection device. As such, in the present text, the distal direction must be understood as the direction of injection with reference to the injection system, and the proximal direction is the opposite direction, i.e., the direction towards the hand of the user.

A medical injection device includes a container for securely storing a medical solution therein. Such a container generally includes an elongated hollow body and a tip extending distally from the body. The tip of the container is provided with a fluid passageway extending through the tip, so as to allow the medical solution in the container to be administered to a patient as necessary. For connection with a needle or with a needleless access device, the tip may be provided with an adaptor comprising internal threads allowing a screwed connection.

Before use, the medical injection device can be prefilled with a medical solution and equipped with a tip cap for closing said passageway, ensuring tightness of the medical container on its distal end, hereby avoiding leakage and/or contamination of the medical solution. For this purpose, screw-on tip caps can be mounted on the adaptor on the distal end of the medical container.

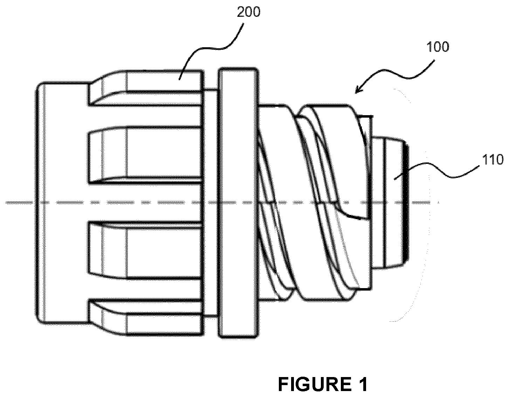

Such screw-on tip caps typically include an inner cap having a skirt made of an elastomeric material, ensuring a tight connection between the tip cap and the medical container, once the tip cap is fastened on the tip of the medical container. Figure 1 illustrates such a tip cap comprising a rigid outer cap 200 and an elastomeric inner cap 100 comprising a skirt 110. The skirt 110 of the inner cap 100 is configured for enclosing the tip of the medical device, and is protruding proximally from the proximal end of the outer cap.

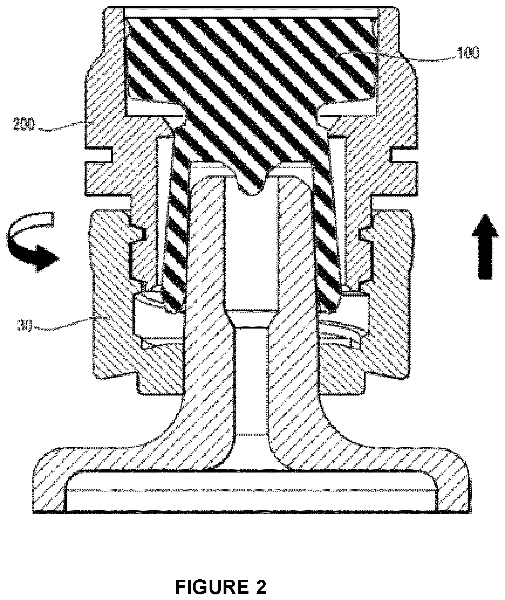

However, the rotative movement of the screw-on process when sealing the medical device frequently leads to a torsion of the elastomer skirt 110, as illustrated by the arrow on the the left side in figure 2. The skirt 110 is hence twisted during the sealing step when the outer cap 200 is screwed on the adaptor and can rotate back in the opposite direction after screwing the tip cap on the tip of the medical container. Hence, a tight screwing might be partially reversed, which leads to a leaking connection and may result in contamination and/or loss of the medical solution.

In other cases, the skirt can be compressed during the mounting of the tip cap, as illustrated by the arrow on the right side in figure 2. After the mounting process, the skirt stretches out along the tip of the medical device in proximal direction to relax its internal constraint and/or torsion. In other cases, due to the friction coefficient and the contact surface between the glass tip of the syringe and the rubber inner cap, a sticking effect may occur, and thus the tip cap requires a high pullout force for removal before use of the medical injection device. Hence, it is difficult for the user to remove the tip cap, especially for a user who might have reduced strength in his fingers due to his medical condition. In the case of healthcare professionals, the repetition of the same gesture requiring to apply a high force onto the tip cap may cause repetitive strain injuries.

Further, the elastomeric skirt is frequently treated with a lubricant for facilitating the mounting process. However, during storage of a plurality of tip caps in a bag, said lubricant may contaminate the surfaces of the rigid parts of the tip cap, in particular the threads of the tip cap to be screwed on the adaptor mounted on the medical container. This can lead to a lubricant contamination of the threads of the adaptor which may imply - when a further needle or connector is screwed onto the adaptor after removing of the tip cap, a bad connection between said connector and the adaptor. Indeed, since lubricant has been deposited onto the threads, the final user may want to stronger screw the connector and hereby accidentally break either the connector or the adaptor.

There is therefore a need for an improved tip cap allowing for tight and secure screwing to the medical container, easy removal of the tip cap before use, and avoiding an unwanted distribution of the lubricant.

Documents US 2019/0046736 A1, US 2022/080129 A1, and US 2028/256880 A1 describe tip caps of prior art.

### SUMMARY OF THE DISCLOSURE

A goal of the present disclosure is to provide an improved tip cap for sealing an injection device, with reduced sticking and torque properties in order to ensure a reliable connection between the tip cap and the injection device.

To that end, an object of the disclosure is a tip cap to close a fluid passageway extending through a distal tip of a medical container, the medical container comprising an adaptor mounted onto the distal tip and having a threaded inner portion, the tip cap comprising:
- an inner cap sealingly engageable with the tip, said inner cap being made of an elastomeric material and comprising a skirt extending in proximal direction, an inner surface of said skirt being configured to sealingly engage an outer surface of the tip of the medical container along a contact surface,
- a rigid outer cap securely disposed around said inner cap, said outer cap comprising a threaded outer portion adapted to engage the threaded inner portion of the adaptor,
wherein the proximal edge of the skirt of the inner cap is aligned with the proximal edge of the outer cap.

Further, the provided tip cap prevents from distributing lubricant on the rigid parts during storage.

Advantageously, the alignment of the proximal end of the skirt of the inner cap and the proximal end of the outer cap presents a mechanical tolerance equal to or less than +/-0.3mm.

Preferably, the proximal edge of the skirt of the inner cap and the proximal end of the rigid outer cap are located in a same plane perpendicular to the tip.

The inner cap may be made of thermoplastic elastomer or rubber.

The rigid outer cap may be made of a transparent rigid plastic material.

A transparent outer cap allows the user to inspect the cap in order to detect a leakage.

Preferably, the rigid outer tip cap is made of a rigid polymer chosen among the list comprising polypropylene, polyethylene, polyvinylchloride, polystyrene, polycarbonate, acrylonitrile butadiene styrene or styrene acrylonitrile.

Advantageously, the inner cap is assembled by frictional engagement and/or clipping on an inner surface of the outer cap.

A second aspect of the invention concerns a tip cap assembly comprising a tip cap as described above, and an adaptor adapted to be engaged with a distal end of a medical container such that said adaptor concentrically surrounds the tip of the medical container, the adaptor comprising a threaded inner portion adapted to engage the threaded outer portion of the outer cap.

A third aspect of the invention concerns a medical injection system comprising:
- a medical container including a longitudinal barrel, a tip projecting distally from the barrel and provided with a fluid passageway extending through the tip, and an adaptor having a threaded inner portion extending around the tip, and
- the tip cap as described above,
wherein the threaded outer portion of the outer cap engages the threaded inner portion of the adaptor and the skirt of the inner cap sealingly engages the tip.

### BRIEF DESCRIPTION OF THE DRAWINGS

Further features and advantages will appear in the following detailed description, based on the appended drawings, wherein:
Figure 1 is a schematic view of a conventional tip cap.
Figure 2 schematically illustrates the forces acting on the skirt of the inner cap in a conventional tip cap.
Figure 3 is a schematic view of a tip cap according to an embodiment of the present disclosure.
Figure 4 is a side view of a syringe with a tip cap assembly according to the invention.
Figure 5 is a schematic view of the elastomeric inner cap of the tip cap shown in figure 2.
Figure 6 is an enlarged view of the tip cap of figure 4.
Figure 7 is a probability plot of the pullout force for a conventional tip cap and a tip cap according to an embodiment.

### DETAILED DESCRIPTION OF EMBODIMENTS

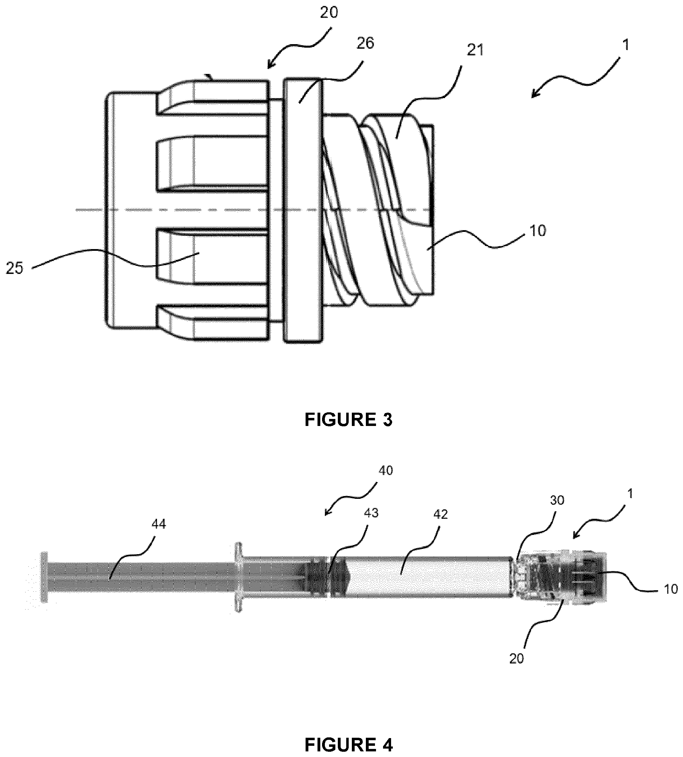

Figure 3 shows a tip cap 1 according to the present invention.

The tip cap 1 can be mounted on a medical injection device 40 such a syringe, as illustrated in figure 4.

The medical injection device 40 includes a container including an elongated hollow body 42 prefilled with a medical solution. A tip 41 extending from said body 42 is provided with a fluid passageway, allowing the medical solution to flow through the tip 41 to be administered to a patient.

Typically, the medical container 40 comprises an adaptor 30 which is mounted on the distal end of the syringe, surrounding at least partially the tip 41 of the syringe. Such an adaptor 30 comprises an inner threaded portion 31, which might also allow to connect a device for administering the medical solution to the body of the patient, such as an injection needle, a device for administering an intravenous infusion, or a needleless injection device.

The tip cap 1 is screwed on the adaptor 30 during storage of the injection device 40, after the container has been filled with the medical solution. The tip cap seals the tip of the medical container in order to prevent leakage and contamination of said medical solution.

The medical injection device can further comprise a plunger rod 44 comprising a stopper 43 which is inserted into the open proximal end of the body 42 and maintaining a fluid-tight engagement with the cylindrical wall of the body 42. After removal of the tip cap 1, a distal sliding movement of the plunger rod 44 urges the medical solution out of the body 42 through the fluid passageway in the tip 41 of the medical injection device 40.

As can be seen in figure 3, the tip cap 1 includes an outer cap 20 and an inner cap 10.

The outer cap 20 is made of a transparent rigid material, preferably a rigid polymer. It can be manufactured by a molding process, for example by injection molding. The polymer can be chosen among polypropylene, polyethylene, polyvinylchloride, polystyrene, polycarbonate, acrylonitrile butadiene styrene, styrene acrylonitrile, or similar polymers, or a combination thereof. The transparent material allows for verifying the correct assembly of the outer cap and the inner cap, and the assembly of the tip cap on the adaptor of the medical container. Further, when the tip cap is mounted on a medical container filled with a medical solution, the transparent outer cap allows the user to inspect the cap and check if leakage has taken place and if medical solution has penetrated into the space between the inner cap and the outer cap. Most preferably, the outer cap is made of polypropylene, as it is an easily moldable, strong and lightweight plastic material. Polypropylene further has the advantages of being highly impermeable to liquids and inert with respect to many chemicals, which prevents degradation of the outer cap when it gets into contact with a medical solution.

The outer cap 20 comprises an outer threaded surface 21 on its proximal end, adapted to be screwed to the inner threads 31 of the adaptor 30 of the medical container 40.

Further, the outer cap 20 can comprise a rim 26 which provides a limiting stop when screwing the tip cap 1 on the adaptor 30, either by contact between the rim 26 and the adaptor 30, or by contact between the inner cap 10 and the distal end of the medical container 40.

The outer cap 20 can further comprise a distal gripping portion 25 comprising ribs, grooves indentations or any other structure allowing the user to securely hold the tip cap 1 when fastening it on the adaptor 30 of a syringe, avoiding slipping of the tip cap in the user's hand. The gripping portion 26 can also prevent the tip cap from rolling away when it is placed onto a smooth surface, making the handling easier to the user.

The outer cap 20 provides the tip cap 1 with a rigid outer wall, in order to protect the elastomeric skirt of the inner cap 10 from mechanical forces due to the external environment, avoiding hereby deformation of the inner cap such as compression or squeezing.

The outer cap 20 can comprise an opening on its distal end which can be of any size smaller or equal to the diameter of the outer cap 20. Such an opening does not affect the function of the outer cap while it simplifies the assembly of the outer cap 20 and the inner cap 10 as it avoids the compression of air between the inner cap and the outer cap, which could create a resistance for inserting the inner cap 10 into the outer cap 20.

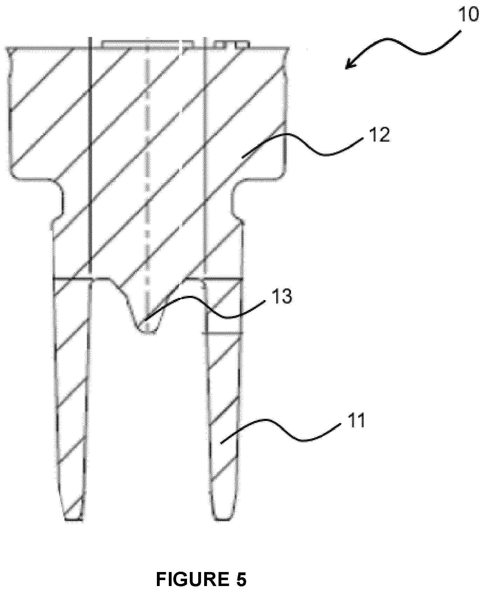

Figure 5 depicts the inner cap 10. Said inner cap 10 is made of an elastomeric material, for example a rubber formulation such as bromobutyl or chlorobutyl elastomers or any other rubber formulation, or a thermoplastic elastomer providing an elasticity suitable for sealing the tip of the medical container. The skilled person will choose a rubber formulation inert to the medical solution to be filled into the medical container, with sufficient heat resistance to undergo the necessary sterilization procedures, and with suitable gripping and releasing properties for slipping the inner cap on the tip of the medical container and releasing it after use. The inner cap 10 can be manufactured by a molding process such as injection molding.

The inner cap 10 comprises a proximal skirt 11 having a hollow cylindrical shape. The inner walls of the hollow cylinder are straight, and the outer walls of the hollow cylinder are slightly tapered. The inner cap further comprises a distal solid sealing base 12 which can present a central protrusion 13 surrounded by said skirt 11, forming an inner cavity having a circular shape adapted to engage the distal edge of the tip of the medical container. The central protrusion 13 can penetrate into the distal end of the tip 41 of the medical container 40.

The diameter of the skirt 11 is chosen so that the skirt is slightly stretched radially when mounted on the tip of the medical container. In this way, the inner wall of the skirt tightly encloses the outer surface of the tip 41 of the medical container 40. The tight cylindrical geometry and the elastomeric material enable the inner cap provide a liquid-tight sealing of the tip of the medical container, avoiding any loss and/or contamination of medical solution.

The inner cap 10 is typically fixed on the inner wall of the outer cap 20 by a clipping mechanism, or any other connection based on a frictional engagement of the inner cap 10 and the outer cap 20. Both the inner cap and the outer cap can comprise ribs 28, grooves 18, or nubs suitable to provide such a connection of the inner cap and the outer cap. Alternatively, the inner cap can be bonded to the outer cap, for example by a suitable glue.

The inner cap 10 and the outer cap 20 are in close contact in a distal portion of the tip cap. At the proximal end of the tip cap, a radial gap 50 is present between the inner cap 10 and the outer cap 20. Said radial gap allows for a deformation of the elastomeric inner cap in order to fit the shape of the tip of a medical device during assembly.

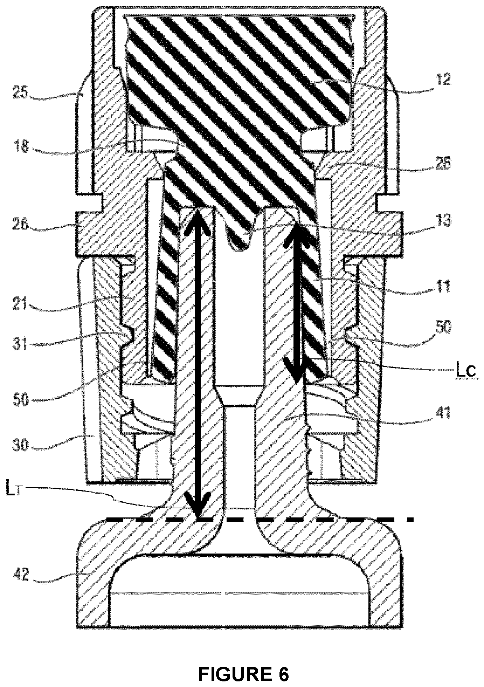

When the outer cap 20 and the inner cap 10 are assembled, as shown in figure 6, the skirt 11 of the inner cap 10 is aligned with the rigid outer cap 20 in proximal direction. The alignment is to be understood that the proximal edge of the skirt of the inner cap and the proximal edge of the outer cap are located in a same plane perpendicular to the tip of the medical device. The tip cap engages the tip of the medical device along a contact surface.

The length of the outer cap has a mechanical tolerance of 0.1 mm, and the length of the skirt of the inner cap has a mechanical tolerance of 0.2 mm. These tolerances are typically due to manufacturing. A total mechanical tolerance of +/-0.3mm is therefore acceptable for the alignment. The edge of the skirt of the inner cap and the end of the outer cap, when assembled, are therefore +/-0.3 mm aligned in proximal direction.

For example, in a tip cap of the type 7025, the skirt has a total length of 5.82 mm. a tolerance of +/- 0.3 mm results in a length variation of 5.2%. In a tip cap of the type FM30, the total cap has a length of 5.77 mm. a tolerance of +/- 0.3 mm results in a length variation of equally 5.2% of the total length of the tip cap.

In a preferred embodiment, the proximal end of the outer cap is flush with the inner cap, i.e., the proximal end of the outer cap and the proximal end of the skirt of the inner cap are precisely located in a same plane perpendicular to the tip of the medical device.

When fixing the tip cap 1 on a prefilled medical container 40, the outer cap 20 is screwed with the external threaded surface 21 into the internal thread 31 of the adaptor 30 mounted on the distal end of a medical container 40. This step is typically performed by a screwing machine. Thereby, the inner cap 10 is pushed in proximal direction, the skirt 10 sliding along the outer surface of the tip 41 and tightly enclosing the tip 41. When the tip cap 1 is mounted, the sealing base 12 seals the fluid passageway on the tip 41 of the medical container, the central protrusion 13 being inserted into the tip 41 of the medical container. The skirt 11 is tightly surrounding the tip 41 in order to provide a seal to the medical solution contained in the medical container 40.

During the screwing procedure, due to its relatively short length, the skirt 11 of the inner cap 10 remains, except for a slight radial stretching, in its original shape. The skirt is neither squeezed nor twisted. The skirt 11 therefore reaches its final shape and position immediately at the end of the screwing, tightly enclosing the tip of the medical container. The tip cap stays in place as screwed on the syringe and does not move or relax into any different position after fixing on the medical container.

### Pullout performance

The tip cap according to the invention presents a contact surface with the tip of the medical container which is smaller than the contact surface of a conventional tip cap in which the skirt of the inner cap extends beyond the rigid outer cap. Preferably, the extension L_{C} of the contact surface in proximal direction is equal to or less than two thirds of the length L_{T} of the distal tip, more preferably equal to or less than half of the length L_{T} of the distal tip. The length L_{T} of the distal tip corresponds to the distance between the distal outer wall of the syringe barrel and the distal end of the tip, as illustrated in figure 6.

This results in a reduction of the pullout force when removing the tip cap from the medical container.

The reduction of the contact surface results in a reduced friction force between the tip cap and the tip of the medical container and hereby reduces the necessary pullout force to be applied in order to remove the tip cap. A reduced pullout force makes it easier for the user to access the medical solution, in particular for a user with reduced force in his fingers, or for a healthcare professional carrying out the same gesture repeatedly.

As the user needs to apply a lower force and less effort, the reduced pullout force and easy removal prevent from accidental breaking the tip in itself, or to break said tip off the medical container. Also, when applying less effort, the risk that the user accidentally twists or bends the tip during removal of the tip cap is reduced. This prevents from breaking the syringe tip, which would make the syringe and the medical solution contained therein useless.

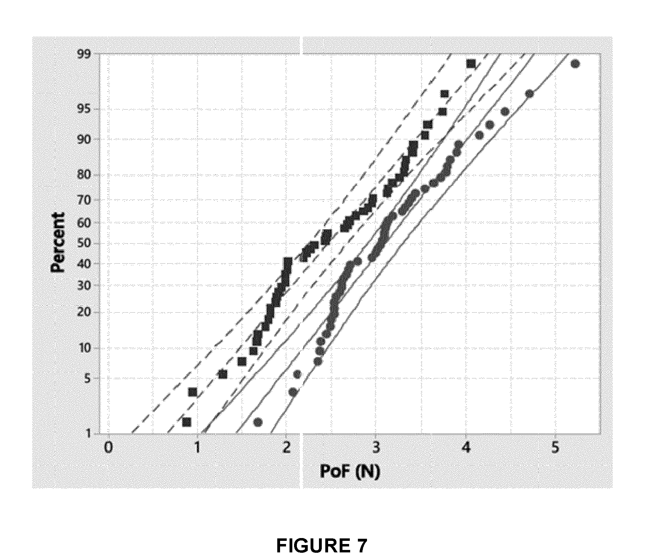

The pullout force has been tested for a range of sample tip caps for different syringe types. Figure 7 shows the distribution of the pullout force in N for two lots of tip caps, one lot of conventional tip caps (represented by circles) and one lot of tip caps according to the invention (represented by squares). The pullout force for the conventional tip caps ranges from 1.6 N to 5.2 N, and the pullout force for the tip caps according to the invention ranges from 0.8 N to 4.1 N. As can be seen in the probability plot, the pullout force is considerably lower for the tip caps according to the invention.

In particular, while providing reduced pullout force, the reduction of the surface does not affect the integrity performances, the tightness of the connection being at the same level as the tightness of a conventional tip cap.

As sterilization and ageing typically affect the pullout force necessary to remove the tip cap, the pullout properties of tip caps according to the invention have been tested after a sterilization procedure and after applying an accelerated ageing protocol.

### Leakage tests

Several lots of tip caps according to the invention have been tested with respect to their sealing performance.

A first type of leakage testing procedure is a combined closure leakage and pressure leakage test, allowing to evaluate the risk of loss of a medical solution contained in the medical container. Therefore, a set of medical containers has been sterilized and filled with a test solution. A closure leak test was carried out, applying a pressure of 1.1 bars during 5 seconds and measuring the leakage rate of the test solution. All samples were in accordance with the specification which requests a leakage rate below 44 Pa/sec.

After the closure leak test, a visual leakage test has been carried out. Subsequently, the medical containers were stoppered with a syringe stopper, leaving a 3-4 mm air bubble as it typically occurs in stoppered medical containers. Then a pressure leak test is performed, during which a pressure is applied to the piston of the syringe stopper and the leakage of test liquid though the tip of the medical container is observed as a function of the applied mechanical pressure. The results were calibrated by subtracting the gliding force of the stopper which was measured separately on an empty injection device. All the recorded pressure leakage values were within the specification of 1.1 bars.

A second type of leakage testing procedure is a dye immersion test, allowing to test penetration of an externally applied substance into the syringe and evaluate the risk of contamination of the medical solution contained therein. For this type of test, a second set of medical containers is sterilized and stoppered with syringe stoppers in empty state. The stopper is positioned at a level which corresponds to a filling level of 1 ml of medical solution in the medical container. Subsequently, the tip cap is mounted on the medical container. During the testing procedure, the tip with the mounted tip cap is immersed in a liquid dye, typically a dye of methylene blue. Thereby, the medical container is not fully immersed. A vacuum of -350mbar is applied for 16h. Penetration of the dye into the medical barrel indicates a leakage and potential risk of contamination from the outside of the tip cap. No leakage has been observed among all the tested samples.

### Distribution of lubricant

Tip caps for medical injection devices are often treated with a lubricant during or after manufacturing and assembly. Such lubricant is typically applied on the elastomeric inner cap in order to facilitate the sliding of the tip cap on the tip of the medical container. During storage, a number of tip caps are typically stored in a clean and dustproof bag, for example a sealed plastic bag. As the elastomeric inner cap is completely surrounded by the outer cap, the lubricant generally remains in place and does not rub off onto the threaded portion of the outer cap nor, consequently, on the threaded portion of the adaptor. As therefore the threaded portion of the adaptor remains free of lubricant, a needle or any other connector screwed on the adaptor after removing the cap will be tightly secured, without risk of loosening the screwed connection. This also prevents from inciting the user to use stronger force for screwing the connector, hereby reducing the risk of accidentally breaking the adaptor or the needle or connector to be screwed on the medical container.

## Claims

1. A tip cap (1) to close a fluid passageway extending through a distal tip of a medical container, the medical container comprising an adaptor mounted onto the distal tip and having a threaded inner portion, the tip cap (1) comprising:
• an inner cap (10) sealingly engageable with the tip, said inner cap (10) being made of an elastomeric material and comprising a skirt (11) extending in proximal direction, an inner surface of said skirt (11) being configured to sealingly engage an outer surface of the tip of the medical container along a contact surface,
• a rigid outer cap (20) securely disposed around said inner cap, said outer cap comprising a threaded outer portion (21) adapted to engage the threaded inner portion of the adaptor,
**characterized in that** the proximal edge of the skirt of the inner cap (10) is aligned with the proximal edge of the outer cap (20).

2. The tip cap (1) according to claim 1, wherein the alignment of the proximal end of the skirt (11) of the inner cap and the proximal end of the outer cap (20) presents a mechanical tolerance equal to or less than +/-0.3mm.

3. The tip cap (1) according to claim 1, wherein the proximal edge of the skirt (11) of the inner cap (10) and the proximal end of the rigid outer cap (20) are located in a same plane perpendicular to the tip.

4. The tip cap (1) according to any of the previous claims, wherein the inner cap (10) is made of thermoplastic elastomer or rubber.

5. The tip cap (1) according to any of the previous claims, wherein the rigid outer cap (20) is made of a transparent rigid plastic material.

6. The tip cap (1) according to any of the previous claims, wherein the rigid outer cap (20) is made of a rigid polymer chosen among the list comprising polypropylene, polyethylene, polyvinylchloride, polystyrene, polycarbonate, acrylonitrile butadiene styrene or styrene acrylonitrile.

7. The tip cap (1) according to any of the previous claims wherein the inner cap (10) is assembled by frictional engagement and/or clipping on an inner surface of the outer cap (20).

8. A tip cap assembly comprising a tip cap (1) according to any of the previous claims, and an adaptor adapted to be engaged with a distal end of a medical container such that said adaptor concentrically surrounds the tip of the medical container, the adaptor comprising a threaded inner portion adapted to engage the threaded outer portion (21) of the outer cap (20).

9. A medical injection system comprising:
- a medical container including a longitudinal barrel, a tip projecting distally from the barrel and provided with a fluid passageway extending through the tip, and an adaptor having a threaded inner portion extending around the tip, and
- the tip cap (1) of any of claims 1 to 8,
wherein the threaded outer portion (21) of the outer cap (20) engages the threaded inner portion of the adaptor and the skirt of the inner cap (10) sealingly engages the tip.

## Patentansprüche

1. Eine Spitzenkappe (1) zum Verschließen eines Fluiddurchgangs, der sich durch eine distale Spitze eines medizinischen Behälters erstreckt, wobei der medizinische Behälter einen auf der distalen Spitze montierten Adapter umfasst, der einen gewindeten Innenabschnitt aufweist, wobei die Spitzenkappe (1) umfasst:
• eine Innenkappe (10), die dicht mit der Spitze verbindbar ist, wobei die Innenkappe (10) aus einem elastomeren Material besteht und eine in proximaler Richtung verlaufende Hülse (11) umfasst, deren innere Oberfläche so ausgestaltet ist, dass sie entlang einer Kontaktfläche dicht mit einer äußeren Oberfläche der Spitze des medizinischen Behälters verbindbar ist,
• eine starre Außenkappe (20), die fest um die Innenkappe angeordnet ist, wobei die Außenkappe einen gewindeten Außenabschnitt (21) umfasst, der dazu geeignet ist, mit dem gewindeten Innenabschnitt des Adapters einzugreifen,
**dadurch gekennzeichnet, dass** die proximale Kante der Hülse der Innenkappe (10) mit der proximalen Kante der Außenkappe (20) fluchtet.

2. Die Spitzenkappe (1) nach Anspruch 1, wobei die Fluchtung des proximalen Endes der Hülse (11) der Innenkappe und des proximalen Endes der Außenkappe (20) eine mechanische Toleranz von gleich oder weniger als +/-0,3 mm aufweist.

3. Die Spitzenkappe (1) nach Anspruch 1, wobei die proximale Kante der Hülse (11) der Innenkappe (10) und das proximale Ende der starren Außenkappe (20) in einer gleichen, zur Spitze senkrechten Ebene liegen.

4. Die Spitzenkappe (1) nach einem der vorhergehenden Ansprüche, wobei die Innenkappe (10) aus thermoplastischem Elastomer oder Gummi besteht.

5. Die Spitzenkappe (1) nach einem der vorhergehenden Ansprüche, wobei die starre Außenkappe (20) aus einem transparenten starren Kunststoffmaterial besteht.

6. Die Spitzenkappe (1) nach einem der vorhergehenden Ansprüche, wobei die starre Außenkappe (20) aus einem starren Polymer besteht, das aus der Liste ausgewählt ist, umfassend Polypropylen, Polyethylen, Polyvinylchlorid, Polystyrol, Polycarbonat, Acrylnitril-Butadien-Styrol oder Styrol-Acrylnitril.

7. Die Spitzenkappe (1) nach einem der vorhergehenden Ansprüche, wobei die Innenkappe (10) durch Reibschluss und/oder Einrasten an einer Innenoberfläche der Außenkappe (20) montiert ist.

8. Eine Spitzenkappenanordnung, umfassend eine Spitzenkappe (1) nach einem der vorhergehenden Ansprüche und einen Adapter, der dazu geeignet ist, mit einem distalen Ende eines medizinischen Behälters einzugreifen, so dass der Adapter konzentrisch die Spitze des medizinischen Behälters umgibt, wobei der Adapter einen gewindeten Innenabschnitt umfasst, der geeignet ist, mit dem gewindeten Außenabschnitt (21) der Außenkappe (20) einzugreifen.

9. Ein medizinisches Injektionssystem umfassend:
- einen medizinischen Behälter, der einen longitudinalen Zylinder, eine sich distal vom Zylinder erstreckende Spitze mit einem sich durch die Spitze erstreckenden Fluiddurchgang und einen Adapter mit einem die Spitze umgebenden gewindeten Innenabschnitt umfasst, und
- die Spitzenkappe (1) nach einem der Ansprüche 1 bis 8,
wobei der gewindete Außenabschnitt (21) der Außenkappe (20) mit dem gewindeten Innenabschnitt des Adapters eingreift und die Hülse der Innenkappe (10) dicht mit der Spitze verbindbar ist.

## Revendications

1. Un capuchon d'extrémité (1) destiné à fermer un passage de fluide s'étendant à travers une extrémité distale d'un récipient médical, le récipient médical comprenant un adaptateur monté sur l'extrémité distale et ayant une portion intérieure filetée, le capuchon d'extrémité (1) comprenant :
• un capuchon intérieur (10) pouvant être mis en prise d'étanchéité avec l'extrémité, ledit capuchon intérieur (10) étant constitué d'un matériau élastomère et comprenant une jupe (11) s'étendant dans une direction proximale, une surface intérieure de ladite jupe (11) étant configurée pour être mise en prise d'étanchéité avec une surface extérieure de l'extrémité du récipient médical le long d'une surface de contact,
• un capuchon extérieur rigide (20) disposé de manière sécurisée autour dudit capuchon intérieur, ledit capuchon extérieur comprenant une portion extérieure filetée (21) adaptée pour être mise en prise avec la portion intérieure filetée de l'adaptateur,
**caractérisé en ce que** le bord proximal de la jupe du capuchon intérieur (10) est aligné avec le bord proximal du capuchon extérieur (20).

2. Le capuchon d'extrémité (1) selon la revendication 1, dans lequel l'alignement de l'extrémité proximale de la jupe (11) du capuchon intérieur et de l'extrémité proximale du capuchon extérieur (20) présente une tolérance mécanique égale ou inférieure à +/- 0,3 mm.

3. Le capuchon d'extrémité (1) selon la revendication 1, dans lequel le bord proximal de la jupe (11) du capuchon intérieur (10) et l'extrémité proximale du capuchon extérieur rigide (20) sont situés dans un même plan perpendiculaire à l'extrémité.

4. Le capuchon d'extrémité (1) selon l'une quelconque des revendications précédentes, dans lequel le capuchon intérieur (10) est constitué d'un élastomère thermoplastique ou de caoutchouc.

5. Le capuchon d'extrémité (1) selon l'une quelconque des revendications précédentes, dans lequel le capuchon extérieur rigide (20) est constitué d'un matériau plastique rigide transparent.

6. Le capuchon d'extrémité (1) selon l'une quelconque des revendications précédentes, dans lequel le capuchon extérieur rigide (20) est constitué d'un polymère rigide choisi parmi la liste comprenant le polypropylène, le polyéthylène, le polychlorure de vinyle, le polystyrène, le polycarbonate, l'acrylonitrile butadiène styrène ou le styrène acrylonitrile.

7. Le capuchon d'extrémité (1) selon l'une quelconque des revendications précédentes, dans lequel le capuchon intérieur (10) est assemblé par mise en prise par friction et/ou encliquetage sur une surface intérieure du capuchon extérieur (20).

8. Un ensemble capuchon d'extrémité comprenant un capuchon d'extrémité (1) selon l'une quelconque des revendications précédentes, et un adaptateur adapté pour être mis en prise avec une extrémité distale d'un récipient médical de telle sorte que ledit adaptateur entoure concentriquement l'extrémité du récipient médical, l'adaptateur comprenant une portion intérieure filetée adaptée pour être mise en prise avec la portion extérieure filetée (21) du capuchon extérieur (20).

9. Un système d'injection médicale comprenant :
- un récipient médical comprenant un cylindre longitudinal, une extrémité s'étendant distalement à partir du cylindre et pourvue d'un passage de fluide s'étendant à travers l'extrémité, et un adaptateur ayant une portion intérieure filetée s'étendant autour de l'extrémité, et
- le capuchon d'extrémité (1) selon l'une quelconque des revendications 1 à 8,
dans lequel la portion extérieure filetée (21) du capuchon extérieur (20) est mise en prise avec la portion intérieure filetée de l'adaptateur et la jupe du capuchon intérieur (10) est mise en prise d'étanchéité avec l'extrémité.
